(19) Europäisches Patentamt
European Patent Office
Office européen des brevets

(11) Numéro de publication : **0 478 416 B1**

(12) # FASCICULE DE BREVET EUROPEEN

(45) Date de publication du fascicule du brevet : **26.01.94 Bulletin 94/04**

(51) Int. Cl.[5] : **A61K 31/505,** A61K 31/53

(21) Numéro de dépôt : **91402472.4**

(22) Date de dépôt : **18.09.91**

(54) **Utilisation des dérivés de la triazine et de la pyrimidine pour l'obtention de médicaments reversant la résistance aux agents anticancéreux et antimalariques.**

(30) Priorité : **27.09.90 FR 9011887**

(43) Date de publication de la demande : **01.04.92 Bulletin 92/14**

(45) Mention de la délivrance du brevet : **26.01.94 Bulletin 94/04**

(84) Etats contractants désignés : **AT BE CH DE DK ES FR GB GR IT LI LU NL SE**

(56) Documents cités :
**FR-A- 2 019 646**
**FR-A- 2 521 560**
**FR-A- 2 524 467**
**FR-A- 2 525 597**
**FR-A- 2 544 315**
**THE LANCET, vol. 2, 15 septembre 1984, pages 594-600; J.P. NEIJT et al.: "Randomised trial comparing two combination chemotherapy regimens (Hexa-CAF VS CHAP-5) in advanced ovarian carcinoma"**
**DIALOG INFORMATION SERVICES, dossier 155: Medline, accession no. 03686565, Derwent Publications Ltd, Londres, GB; B.L. JOHNSON et al.: "Hexamethylmelamine in alkylating agent-resistant ovarian carcinoma", & CANCER, NOVEMBRE 1978, 42(5), P2157-61**

(73) Titulaire : **ADIR ET COMPAGNIE**
**1 rue Carle Hébert**
**F-92415 Courbevoie Cédex (FR)**

(72) Inventeur : **Paramelle, Bernard**
**La Cédraie - Avenue des Cèdres**
**F-38700 La Tronche (FR)**
Inventeur : **Leverve, Xavier**
**40 Grand-rue**
**F-38700 La Tronche (FR)**
Inventeur : **Regnier, Gilbert**
**Demeure du Plessis, Bât. D, 27 avenue du Plessis**
**F-92290 Chatenay Malabry (FR)**
Inventeur : **Dhainaut, Alain**
**7 rue des Guipières**
**F-78400 Chatou (FR)**
Inventeur : **Atassi, Ghanem**
**4 rue Joséphine**
**F-92400 Saint Cloud (FR)**
Inventeur : **Pierre, Alain**
**52 rue de Montval**
**F-78160 Marly le Roi (FR)**

(74) Mandataire : **Reverbori, Marcelle et al**
**Adir et Compagnie, 1, rue Carle Hébert**
**F-92415 Courbevoie Cédex (FR)**

EP 0 478 416 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne l'utilisation de dérivés de la triazine et de la pyrimidine pour l'obtention de médicaments reversant la résistance aux agents anticancéreux et antimalariques.

Plusieurs dérivés de la triazine et de la pyrimidine, doués de propriétés pharmacologiques intéressantes, sont connus dans la littérature. Certains parmi ces composés, favorisent la captation d'oxygène et sont utilisés dans le traitement du déclin cérébral, (Brevets FR 2.525.597, FR 2.521.560 et FR 2.524.467).

D'autres trouvent leurs applications dans le traitement de l'insuffisance respiratoire (Brevet FR 2.019.646) ou de tout type d'hypoxie tissulaire (Bevet FR 2.544.315).

La demanderesse a maintenant trouvé que certains de ces dérivés possèdent d'autres propriétés pharmacologiques très intéressantes. En effet, ils reversent partiellement ou totalement la résistance acquise aux agents anticancéreux et aux médicaments antimalariques.

La résistance aux agents anticancéreux est un obstacle majeur à l'efficacité des drogues antitumorales. Les cellules tumorales lorsqu'elles sont exposées in vitro ou in vivo à un agent anticancéreux, deviennent résistantes, à divers degrés à ces composés.

De nombreux mécanismes d'acquisition par une cellule de résistance aux agents anticancéreux ont été décrits. Parmi les différents types de résistance, la "Multidrug Résistance" (MDR) est particulièrement intéressante. Le phénomène de résistance est dû à l'action d'une protéine membranaire inductible, la gP 170, dont le rôle est d'augmenter l'efflux du cytotoxique, donc de diminuer sa concentration intracellulaire, d'où la perte de sensibilité de ces cellules à la drogue.

Des médicaments, utilisés dans d'autres pathologies, sont connus pour reverser, partiellement ou totalement cette résistance (Int. J. Cancer Res. (1988) 79 pp 285-296 ; J.N.C.I. (1989) 81 pp 907-910 ; Trends Pharmacol. Sci. (1989) 9 pp 54-58 ; Annu. Rev. Biochem. (1989) 58 pp 137-171).

L'agent modulateur, lorsqu'il est ajouté en même temps que le cytotoxique diminue ou supprime totalement la résistance de type MDR. Certains médicaments utilisés pour le traitement d'autres maladies comme l'amiodarone, le verapamil ou la ciclosporine, ont été utilisés en clinique pour lever cette résistance, mais leurs propriétés pharmacologiques intrinsèques (agents hypotenseurs ou immunosuppresseurs) souvent indésirables lors du traitement du cancer et leurs toxicités, limitent considérablement leur utilisation.

Le mécanisme de la résistance à la chloroquine, developpée par le Plasmodium falciparum est similaire. Le verapamil restaure la sensibilité d'une lignée résistante, ce qui démontre l'intérêt potentiel de composés reversant le phénotype MDR des cellules tumorales, pour une utilisation en parasitologie. (Science (1987), 238, pp 1283-1285 ; Science (1987), 235, pp 899-901). L'état antérieur de la technique le plus proche de la présente invention est illustré par : The Lancet, 2, (1984), 594-600, et Cancer, 42(5), (1978), 2157-61, qui mentionnent entre autres l'utilisation de l'hexaméthylmélamine pour réverser la résistance aux agents anti-tumoraux.

La présente invention a particulièrement pour objet l'utilisation, pour l'obtention de médicaments reversant la résistance acquise aux agents anticancéreux et antimalariques, des composés de formule I :

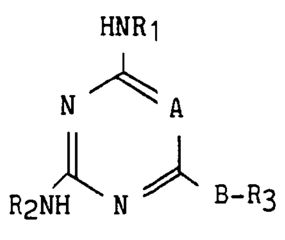

(I)

dans laquelle

- **A** représente un groupe -CH- ou un atome d'azote,
- **$R_1$ et $R_2$** identiques ou différents représentent chacun un radical hydrocarboné ayant 3 à 5 atomes de carbone en chaine droite ou ramifiée renferment éventuellement une ou deux double liaisons et éventuellement substitué par un ou plusieurs radicaux hydroxyles,
- **B** représente un radical de formule $y_1$ :

(y1)

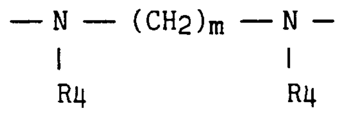

(dans laquelle $R_4$ représente un atome d'hydrogène ou un radical alkyle ayant de 1 à 5 atomes de carbone, et m représente un nombre entier de 2 à 6),
un radical de formule $y_2$ :

(y2)

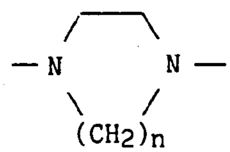

(dans laquelle n représente les nombres entiers 2 ou 3),
un radical de formule $y_3$ :

(y3)

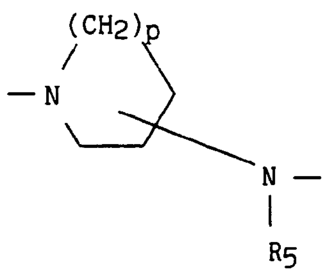

(dans laquelle p représente zéro ou les nombres entiers 1 ou 2,
et
- $R_5$ représente un atome d'hydrogène, un radical alkyle ayant de 1 à 5 atomes de carbone, un radical cycloalkyle ayant de 3 à 7 atomes de carbone),

ou un radical de formule $y_4$ :

(y4)

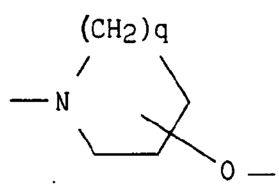

(dans laquelle q représente zéro ou les nombres entiers 1 ou 2)
- **$R_3$** représente un radical diphénylméthyle, éventuellement substitué sur les cycles benzéniques par un ou plusieurs atomes d'halogène,

un radical de formule $z_1$ :

(z1)

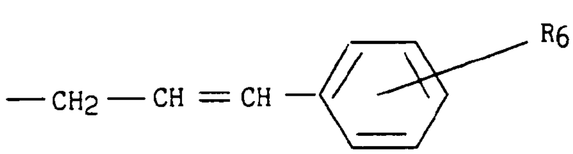

(dans laquelle $R_6$ représente un atome d'hydrogène ou un atome d'halogène), un radical de formule $z_2$ :

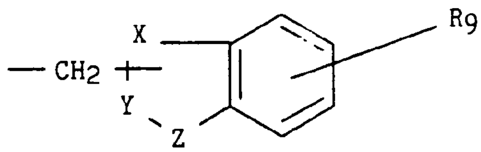

(z2)

(dans laquelle X-Y- représente un reste de formule :

$$-CH=CH-(CR_7R_8)_r-$$

dans laquelle r représente 0 ou 1 et $R_7$, $R_8$ identiques ou différents représentent chacun un atome d'hydrogène ou un radical méthyle, Z représente un atome d'oxygène ou de soufre et $R_9$ représente un atome d'hydrogène ou un atome d'halogène),

ainsi que leurs sels d'addition obtenus avec un acide minéral ou organique thérapeutiquement compatible. les composés de formule I sont structurellement bien différenciés de l'hexaméthylmélamine, puisque même lorsque A représente un atome d'azote, le noyau triazine n'est jamais trisubstitué par un radical diméthylamino et renferme toujours le substituant -B-$R_3$ nullement suggéré par l'enseignement de l'état de la technique.

Comme acides utilisés pour la formation des sels d'addition, on peut citer l'acide chlorhydrique, sulfurique, bromhydrique, phosphorique, acétique, propionique, maléique, benzoïque, méthanesulfonique.

Les médicaments obtenus en utilisant selon l'invention les composés de formule I ou leurs sels pharmaceutiquement acceptables, seront présentés sous des formes pharmaceutiques convenant pour l'administration par voie orale, parentérale per ou transcutanée comme par exemple comprimés, gélules, tablettes, solutions injectables ou buvables.

La posologie peut varier largement selon l'âge et le poids du patient, la voie d'administration, la nature de la maladie et des traitements associés, et s'échelonne de 0,10 à 7 g par prise.

Les activités pharmacologiques des composés de formule I décrites dans les brevets FR 2.525.597, FR 2.521.560, FR 2.524.467, Fr 2.019.646 et FR 2.544.315, ne laissaient présager en aucun cas les propriétés fort intéressantes découvertes par la demanderesse. Toutefois, les activités antérieurement connues, ne constituent pas un facteur limitant de l'utilisation des composés de formule I lors d'un traitement anticancéreux ou antimalarique. Par ailleurs, la toxicité des composés de formule I est très petite ce qui permet l'administration des doses très élévées.

A titre d'exemple des composés de formule I, on peut citer les produits suivants :

<u>Composé 1</u> :

Difumarate de la 1-(4,6-bis allylamino 1,3,5-triazin-2-yl) 3-bis parafluorobenzhydrylamino pipéridine.

<u>Composé 2</u> :

Difumarate de la 1-(4,6-bis allylamino 1,3,5-triazin-2-yl) 4-(benzofuran-2-yl méthyl amino) pipéridine.

<u>Composé 3</u> :

1-(4,6-bis allylamino 1,3,5-triazin-2-yl) 4-(5-fluro benzofuran-2-yl méthyl) pipérazine.

<u>Composé 4</u> :

Chlorhydrate de la 1-(4,6-bis allylamino 1,3,5-triazin-2-yl) 4-(bis parafluorobenzhydryloxy) pipéridine.

<u>Composé 5</u> :

Fumarate de la N,N'-diéthyl N-(4,6-bis allylamino 1,3,5-triazin-2-yl) N'-(benzothien-2-yl méthyl) éthylène diamine.

<u>Composé 6</u> :

Difumarate de la 1-(4,6-bis allylamino 1,3,5-triazin-2-yl) 4-(N-bis parafluorobenzhydryl N-éthyl amino) pipéridine.

Composé 7 :

Difumarate de la 1-(4,6-bis allylamino 1,3,5-triazin-2-yl) 4-(bis parafluorobenzhydrylamino) pipéridine.

Composé 8 :

Difumarate de la 1-(4,6-bis allylamino 1,3,5-triazin-2-yl) 4-(benzothien-2-yl méthyl amino) pipéridine.

Composé 9 :

Dichlorhyrate de la 1-(4,6-bis allylamino 1,3,5-triazin-2-yl) 4-(parafluorobenzhydrylamino) pipéridine.

Composé 10 :

Difumarate de la 1-(4,6-bis allylamino 1,3,5-triazin-2-yl) 4-benzhydrylamino pipéridine.

Composé 11 :

Méthanesulfonate de la 1-(4,6-bis allylamino 1,3,5-triazin-2-yl) 4-(parafluorobenzhydryl) pipérazine.

Les exemples suivants illustrent l'invention.

**EXEMPLE 1**

**Evaluation de l'augmentation de la cytotoxicité de l'adriamycine sur la lignée P 388/ADR-1 in vitro**

Lors de cette étude, on a mesuré la cytotoxicité de l'adriamycine, en absence et en présence du composé reversant. Pour cet essai, on a utilisé la leucémie murine P 388/ADR-1 dont la résistance a été induite par l'adriamycine. Son facteur de résistance est de 40 par rapport à la lignée sensible (résistance moyenne).

Les cellules sont cultivées dans un milieu de culture (RPMI 1640) complet, contenant 10 % de sérum de veau foetal, 2 nM de glutamine, 50 UI/ml de pénicilline, 50 µg/ml de streptomycine, 10 mM de Hepes et 20 nM de bêta-mercaptoéthanol.

Les cellules sont réparties dans des microplaques et exposées à l'adriamycine à 9 concentrations différentes.

Les produits testés pour leur capacité à reverser la MDR sont ajoutés en même temps que le cytotoxique. Les cellules sont ensuite incubées pendant 48 heures.

Le nombre de cellules viables est ensuite quantifié par un essai colorimétrique, le Microculture Tetrazolium Assay (Cancer Res. (1987), 47, pp 936-942).

Les résultats sont exprimés en $IC_{50}$, concentration en cytotoxique qui inhibe à 50 % la prolifération des cellules témoins. Les résultats sont exprimés en Facteur de Réversion (FR).

$$FR = \frac{IC_{50}\ \text{Cytotoxique seul}}{IC_{50}\ \text{Cytotoxique en presence du composé reversant}}$$

Le tableau I donne les valeurs des facteurs de reversions obtenus avec les différents composés de formule I et les produits de référence, et démontre l'activité très intéressante des composés de formule I.

Concernant la reserpine (un des produits de référence), ce composé a une très bonne activité in vitro mais in vivo, il est inutilisable à cause de sa grande toxicité.

TABLEAU I

| COMPOSES | 2,5 µM | 5 µM | 10 µM | 20 µM |
|---|---|---|---|---|
| **PRODUITS DE REFERENCE** | | | | |
| PHENOTHIAZINE | --- | 0,5 | 0,5 | 0,4 |
| CHLOROPROMAZINE | --- | 2,6 | 3,8 | TOX |
| YOHIMBINE | --- | 0,4 | 0,9 | 2,1 |
| NIFEDIPINE | --- | 1,0 | 2,2 | 2,1 |
| PROGESTERONE | --- | 0,6 | 0,7 | 2,1 |
| QUININE | --- | 0,8 | 2,0 | 2,1 |
| DILTIAZEM | --- | 3,1 | 6,1 | 6,5 |
| FLUNARIZINE | --- | 1,5 | 2,9 | 6,9 |
| DIPYRIDAMOLE | --- | 2,2 | 4,2 | 8,5 |
| QUINIDINE | --- | 0,8 | 2,5 | 3,5 |
| QUINACRINE | --- | 3,4 | TOX* | TOX |
| TRIFLUOPERAZINE | --- | 2,9 | TOX | TOX |
| VERAPAMIL | --- | 7,5 | 10,7 | 14,5 |
| AMIODARONE | --- | 8,1 | 10,6 | TOX |
| PIMOZIDE | 7,9 | 19,3 | TOX | TOX |
| RESERPINE | 41 | 42 | 35 | TOX |
| CICLOSPORINE | --- | 21 | 23 | TOX |
| **COMPOSES DE FORMULE I** | | | | |
| COMPOSE 6 | 24,4 | 27,0 | 20,7 | 41,1 |
| COMPOSE 7 | 10,3 | 17,3 | 40,9 | --- |
| COMPOSE 9 | 6,9 | 16,3 | 51,2 | --- |
| COMPOSE 10 | 12,4 | 27,3 | 26,7 | --- |
| COMPOSE 11 | 11,5 | 24,3 | 34,5 | 144 |

* Les composés sont considérés comme toxiques lorsque la toxicité cellulaire est ≥ 50 %

## EXEMPLE 2

**Evaluation de l'augmentation de la cytotoxicité de l'actinomycine D sur la lignée de poumon de hamster chinois, DC-3F/AD**

Le protocole utilisé pour cette étude est identique à celui utilisé pour l'essai décrit dans l'exemple 1 mais le milieu de culture ne contenait pas de bêta-mercaptoéthanol et les cellules ont été incubées pendant 4 jours au lieu de 48 heures. Le cytotoxique utilisé était l'actinomycine D.

La lignée DC-3F/AD est une lignée extrêmement résistante. Son facteur de résistance est supérieur à 10.000.

Les résultats de cette étude sont donnés dans le tableau II :

Les résultats du tableaux II démontrent que les composés de formule I diminuent de manière significative ou suppriment la résistance au cytotoxique.

TABLEAU II

| COMPOSES | 2,5 µM | 5 µM | 10 µM | 20 µM |
|---|---|---|---|---|
| **PRODUITS DE REFERENCE** | | | | |
| PHENOTHIAZINE | --- | < 12 | < 12 | < 12 |
| CHLOROPROMAZINE | --- | < 13 | < 13 | < 13 |
| YOHIMBINE | --- | < 12 | < 12 | < 12 |
| NIFEDIPINE | --- | < 11 | TOX | TOX |
| PROGESTERONE | --- | < 10 | < 10 | 13 |
| QUININE | --- | < 10 | < 10 | < 11 |
| DILTRIAZEM | --- | < 11 | < 10 | < 11 |
| FLUNARIZINE | --- | < 13 | 16 | TOX |
| DIPYRIDAMOLE | --- | < 12 | < 11 | < 12 |
| QUINIDINE | --- | < 12 | < 12 | < 12 |
| QUINACRINE | --- | < 13 | < 12 | TOX |
| TRIFLUOPERAZINE | --- | < 12 | 38 | TOX |
| VERAPAMIL | --- | < 13 | 31 | 117 |
| AMIODARONE | --- | 365 | 276 | TOX |
| PIMOZIDE | < 10 | 28 | 1649 | TOX |
| RESERPINE | 258 | 1085 | < 11 | 2024 |
| CICLOSPORINE | < 11 | < 11 | 23 | 10 |
| **COMPOSES DE FORMULE I** | | | | |
| COMPOSE 1 | < 18 | 118 | 263 | 2283 |
| COMPOSE 2 | --- | 450 | 451 | 2148 |
| COMPOSE 3 | --- | 340 | 831 | 2189 |
| COMPOSE 4 | -- | 290 | 970 | 3302 |
| COMPOSE 5 | < 15 | < 15 | 1013 | 4059 |
| COMPOSE 6 | --- | --- | 1093 | 3744 |
| COMPOSE 7 | --- | 388 | 1212 | 3056 |
| COMPOSE 8 | --- | 310 | 1281 | 7327 |
| COMPOSE 9 | --- | 732 | 1796 | 13657 |
| COMPOSE 10 | --- | 905 | 1828 | 3313 |

**EXEMPLE 3**

**Cytométrie en flux**

Certains composés anticancéreux comme l'adriamycine (ADR) ont la propriété d'être fluorescents après excitation par une source lumineuse de longueur d'onde connue.

Par la mesure de cette fluorescence, il est possible de mesurer de façon relative la concentration intracellulaire en ADR. La cytométrie en flux (CMF) est un outil de choix pour effectuer ce type de mesure et ainsi déterminer rapidement si certains composés actifs agissent en augmentant la concentration intracellulaire en adriamycine.

Les cellules ($500.10^3$) par ml ont été exposées simultanément à l'adriamycine à une concentration fixe (50 µM) et aux composés testés, aux concentrations de 2,5, 10 et 20 µM. Après 5 heures d'incubation, l'uptake de l'adriamycine intra-cellulaire a été évalué par CMF.

Les analyses ont été réalisées sur un cytomètre en flux ATC 3000 (Bruker - France) équipé d'un laser argon 2025 (Spectra-Physics-France®) optimisé à 488 nm pour une puissance de 600 mW.

L'analyse de chacun des échantillons a été effectuée sur un total de 10000 cellules à une vitesse de 1000 cellules/sec.

Les résultats ont été collectés sous forme d'histogrammes linéaires de la fluorescence de l'ADR intra-cellulaire.

**Expression des résultats :**

Pour chacun des histogrammes, le canal moyen (MEAN) de fluorescence a été déterminé par le système informatique de l'appareil.

Pour toutes expériences :

- Un contrôle négatif (cellules sans ADR) a fixé le seuil d'auto-fluorescence.
- Un contrôle positif (cellules avec ADR) a déterminé la valeur MEAN = MN1.
- Les tubes "tests" (cellules avec ADR et avec produit) ont déterminé pour chacun des produits et à chacune des concentrations, les valeurs MEAN = MN2.
- Les résultats sont exprimés sous forme de variation de la moyenne de fluorescence obtenue pour chacun des tubes "tests" (MN2) par rapport à la moyenne de la fluorescence obtenue avec le contrôle positif (MN1) : VAR-MEAN = MN2 - MN1. Le paramètre exprimé est donc l'augmentation de la fluorescence de l'adriamycine en présence des composés testés.

Le tableau III donne l'augmentation de la fluorescence de l'ADR obtenue avec les différents composés sur la lignée DC-3F/AD et le tableau IV sur la lignée P 388/ADR-1.

TABLEAU III

| COMPOSES | 2,5 µM | 5 µM | 10 µM | 20 µM |
|---|---|---|---|---|
| **PRODUITS DE REFERENCE** | | | | |
| VERAPAMIL | 2,00 | 2,60 | 7,55 | 11,45 |
| AMIODARONE | 8,80 | 16,65 | 21,55 | 26,35 |
| PIMOZIDE | 5,85 | 8,85 | 15,65 | 21,60 |
| RESERPINE | 26,18 | 29,43 | 31,73 | 29,95 |
| CICLOSPORINE | 1,90 | 3,20 | 8,05 | 15,10 |
| **COMPOSES DE FORMULE I** | | | | |
| COMPOSE 2 | 1,60 | 2,70 | 23,00 | 20,30 |
| COMPOSE 4 | 4,60 | 11,40 | 16,10 | 20,60 |
| COMPOSE 6 | 31,30 | 42,70 | 44,70 | 52,10 |

TABLEAU IV

| COMPOSES | 2,5 µM | 5 µM | 10 µM | 20 µM |
|---|---|---|---|---|
| **PRODUITS DE REFERENCE** | | | | |
| PHENOTHIAZINE | 0,00 | 0,00 | 0,00 | 0,00 |
| CHLOROPROMAZINE | 3,30 | 5,15 | 4,60 | 6,20 |
| YOHIMBINE | 2,30 | 0,05 | 4,70 | 3,45 |
| NIFEDIPINE | 0,00 | 0,80 | 5,95 | 3,40 |
| PROGESTERONE | 1,85 | 1,00 | 6,85 | 11,80 |
| QUININE | 4,85 | 5,40 | 12,00 | 18,90 |
| DILTIAZEM | 3,10 | 6,65 | 13,45 | 23,15 |
| FLUNARIZINE | 7,35 | 10,10 | 19,90 | 41,00 |
| DIPYRIDAMOLE | 2,35 | 7,55 | 21,75 | 40,00 |
| QUINIDINE | 6,65 | 12,80 | 22,00 | 29,90 |
| QUINACRINE | 11,83 | 15,73 | 27,73 | 52,77 |
| TRIFLUOPERAZINE | 11,45 | 14,75 | 34,55 | 51,05 |
| VERAPAMIL | 9,89 | 21,90 | 39,62 | 56,50 |
| AMIODARONE | 33,40 | 65,25 | 76,33 | 95,85 |
| PIMOZIDE | 25,60 | 49,42 | 73,13 | 73,64 |
| RESERPINE | 73,46 | 80,48 | 91,90 | 85,39 |
| CICLOSPORINE | 79,43 | 97,63 | 96,68 | 90,27 |
| **COMPOSES DE FORMULE I** | | | | |
| COMPOSE 2 | 28,45 | 45,30 | 65,20 | 70,20 |
| COMPOSE 4 | 55,20 | 77,10 | 84,06 | 94,70 |
| COMPOSE 6 | 73,75 | 88,65 | 92,00 | 95,50 |
| COMPOSE 7 | 43,55 | 66,20 | 82,48 | 91,70 |
| COMPOSE 8 | 23,75 | 43,10 | 67,05 | 71,40 |
| COMPOSE 9 | 50,10 | 69,65 | 81,65 | 83,90 |
| COMPOSE 10 | 47,75 | 68,90 | 83,03 | 85,00 |
| COMPOSE 11 | 30,15 | 70,60 | 82,86 | --- |

Les tableaux III et IV démontrent que les composés de formule I augmentent la concentration intracellulaire de l'adriamycine et ils sont aussi actifs que la ciclosporine et l'amiodarone.

**EXEMPLE 4**

**Composition pharmaceutique**

Gélules dosées à 200 mg de principe actif.

| | |
|---|---|
| Composé 11 | 200 mg |
| Amidon de maïs | 50 mg |
| Lactose | 100 mg |
| Talc | 30 mg |

## Revendications

**Revendications pour les Etats contractants suivants : AT, BE, CH, DE, DK, FR, GB, IT, LI, LU, NL, SE**

**1.** Utilisation pour l'obtention de médicaments reversant la résistance acquise aux agents anticancéreux et antimalariques de composés de formule I :

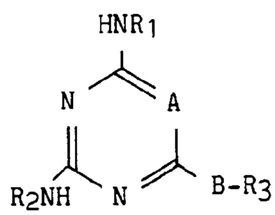

(I)

dans laquelle

- **A** représente un groupe -CH- ou un atome d'azote,
- **$R_1$ et $R_2$** identiques ou différents représentent chacun un radical hydrocarboné ayant 3 à 5 atomes de carbone en chaine droite ou ramifiée renferment éventuellement une ou deux double liaisons et éventuellement substitué par un ou plusieurs radicaux hydroxyles,
- **B** représente un radical de formule $y_1$:

(y1)

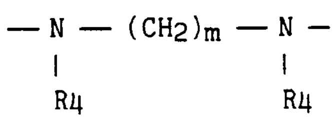

(dans laquelle $R_4$ représente un atome d'hydrogène ou un radical alkyle ayant de 1 à 5 atomes de carbone, et m représente un nombre entier de 2 à 6),
un radical de formule $y_2$ :

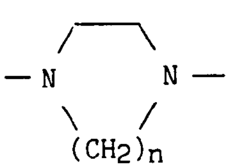

(y2)

(dans laquelle n représente les nombres entiers 2 ou 3),
un radical de formule $y_3$ :

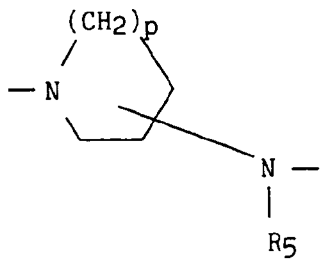

(y3)

(dans laquelle p représente zéro ou les nombres entiers 1 ou 2,
et

- $R_5$ représente un atome d'hydrogène, un radical alkyle ayant de 1 à 5 atomes de carbone, un radical cycloalkyle ayant de 3 à 7 atomes de carbone),
ou un radical de formule $y_4$ :

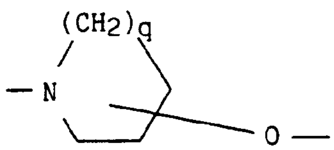

(y4)

(dans laquelle q représente zéro ou les nombres entiers 1 ou 2)

- **$R_3$** représente un radical diphénylméthyle, éventuellement substitué sur les cycles benzéniques par un ou plusieurs atomes d'halogène,

un radical de formule $z_1$ :

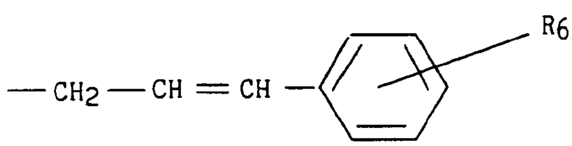

($z_1$)

(dans laquelle $R_5$ représente un atome d'hydrogène ou un atome d'halogène), un radical de formule $z_2$ :

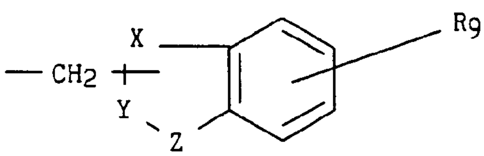

(z2)

(dans laquelle X-Y- représente un reste de formule :

$$-CH=CH-(CR_7\ R_5)_r-$$

dans laquelle r représente 0 ou 1 et $R_7$ $R_8$ identiques ou différents représentent chacun un atome d'hydrogène ou un radical méthyle, Z représente un atome d'oxygène ou de soufre et $R_9$ représente un atome d'hydrogène ou un atome d'halogène),

ainsi que leurs sels d'addition obtenus avec un acide minéral ou organique thérapeutiquement compatible.

**Revendications pour l'Etat contractant suivant : ES**

**1.** Utilisation pour l'obtention de médicaments reversant la résistance acquise aux agents anticancéreux et antimalariques de composés de formule I :

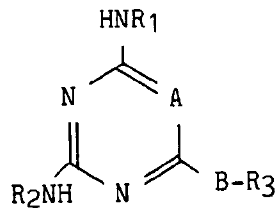

(I)

dans laquelle

- **A** représente un groupe -CH- ou un atome d'azote,
- **$R_1$ et $R_2$** identiques ou différents représentent chacun un radical hydrocarboné ayant 3 à 5 atomes de carbone en chaine droite ou ramifiée renferment éventuellement une ou deux double liaisons et éventuellement substitué par un ou plusieurs radicaux hydroxyles,
- **B** représente un radical de formule $y_1$ :

(y1)

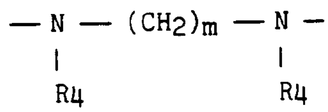

(dans laquelle $R_4$ représente un atome d'hydrogène ou un radical alkyle ayant de 1 à 5 atomes de carbone, et m représente un nombre entier de 2 à 6),
un radical de formule $y_2$ :

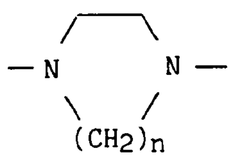

(y2)

(dans laquelle n représente les nombres entiers 2 ou 3),
un radical de formule $y_3$ :

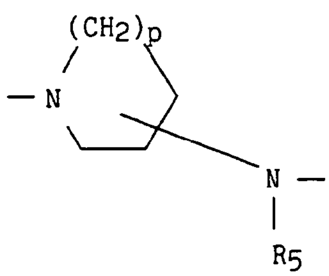

(y3)

(dans laquelle p représente zéro ou les nombres entiers 1 ou 2,
et

- $R_5$ représente un atome d'hydrogène, un radical alkyle ayant de 1 à 5 atomes de carbone, un radical cycloalkyle ayant de 3 à 7 atomes de carbone),

ou un radical de formule $y_4$ :

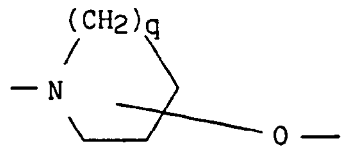

(y4)

(dans laquelle q représente zéro ou les nombres entiers 1 ou 2)

- **$R_3$** représente un radical diphénylméthyle, éventuellement substitué sur les cycles benzéniques par un ou plusieurs atomes d'halogène,

un radical de formule $z_1$ :

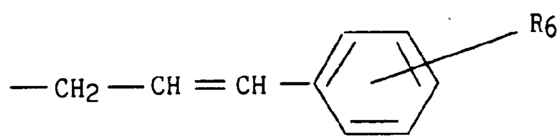

($z_1$)

(dans laquelle $R_6$ représente un atome d'hydrogène ou un atome d'halogène), un radical de formule $z_2$ :

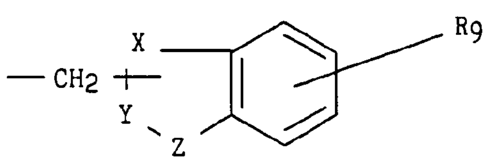

($z_2$)

(dans laquelle X-Y- représente un reste de formule :

$$-CH=CH-(CR_7\ R_6)_r-$$

dans laquelle r représente 0 ou 1 et $R_7$ $R_8$ identiques ou différents représentent chacun un atome d'hydrogène ou un radical méthyle, Z représente un atome d'oxygène ou de soufre et $R_9$ représente un atome d'hydrogène ou un atome d'halogène),

ainsi que leurs sels d'addition obtenus avec un acide minéral ou organique thérapeutiquement compatible.

**Revendications pour l'Etat contractant suivant : GR**

1. Utilisation pour l'obtention de médicaments reversant la résistance acquise aux agents anticancéreux et antimalariques de composés de formule I :

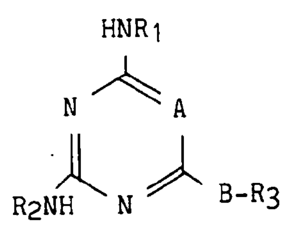

(I)

dans laquelle

- **A** représente un groupe -CH- ou un atome d'azote,
- **$R_1$ et $R_2$** identiques ou différents représentent chacun un radical hydrocarboné ayant 3 à 5 atomes de carbone en chaine droite ou ramifiée renferment éventuellement une ou deux double liaisons et éventuellement substitué par un ou plusieurs radicaux hydroxyles,
- **B** représente un radical de formule $y_1$ :

(y1)

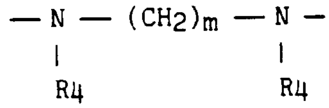

(dans laquelle $R_4$ représente un atome d'hydrogène ou un radical alkyle ayant de 1 à 5 atomes de carbone, et m représente un nombre entier de 2 à 6),
un radical de formule $y_2$ :

(y2)

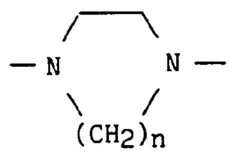

(dans laquelle n représente les nombres entiers 2 ou 3),
un radical de formule $y_3$ :

(y3)

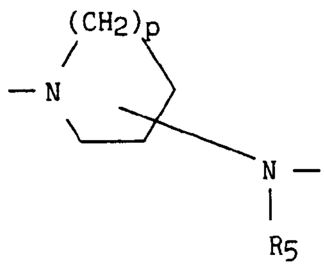

(dans laquelle p représente zéro ou les nombres entiers 1 ou 2,
et

- $R_5$ représente un atome d'hydrogène, un radical alkyle ayant de 1 à 5 atomes de carbone, un radical cycloalkyle ayant de 3 à 7 atomes de carbone),
ou un radical de formule $y_4$ :

(y4)

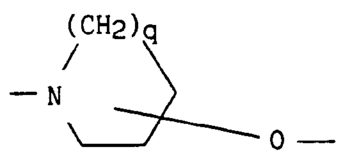

(dans laquelle q représente zéro ou les nombres entiers 1 ou 2)

- **$R_3$** représente un radical diphénylméthyle, éventuellement substitué sur les cycles benzéniques par un ou plusieurs atomes d'halogène,

un radical de formule $z_1$ :

($z_1$)

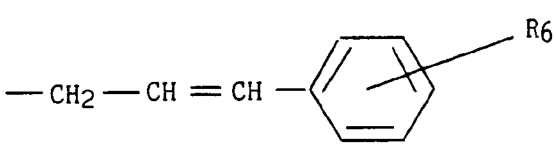

(dans laquelle $R_6$ représente un atome d'hydrogène ou un atome d'halogène), un radical de formule $z_2$ :

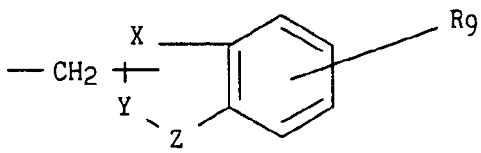

($z_2$)

(dans laquelle X-Y- représente un reste de formule :

$$-CH=CH-(CR_7\ R_6)_r-$$

dans laquelle r représente 0 ou 1 et $R_7$ $R_8$ identiques ou différents représentent chacun un atome d'hydrogène ou un radical méthyle, Z représente un atome d'oxygène ou de soufre et $R_9$ représente un atome d'hydrogène ou un atome d'halogène),

ainsi que leurs sels d'addition obtenus avec un acide minéral ou organique thérapeutiquement compatible.

## Patentansprüche

### Patentansprüche für folgende Vertragsstaaten : AT, BE, CH, DE, DR, FR, GB, IT, LI, LU, NL, SE

1. Verwendung von Verbindungen der Formel I:

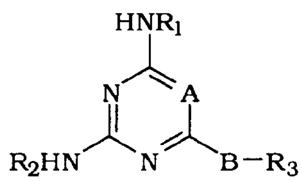

(I)

in der

- A eine Gruppe -CH- oder ein Stickstoffatom,
- $R_1$ und $R_2$, die gleichartig oder verschieden sein können, jeweils eine geradkettige oder verzweigte Kohlenwasserstoffgruppe mit 3 bis 5 Kohlenstoffatomen, die gegebenenfalls eine oder zwei Doppelbindungen aufweisen und gegebenenfalls durch eine oder mehrere Hydroxylgruppen substituiert sein kann,
- B eine Gruppe der Formel $y_1$:

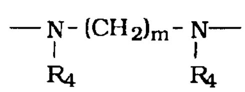

($y_1$)

(worin $R_4$ ein Wasserstoffatom oder eine Alkylgruppe mit 1 bis 5 Kohlenstoffatomen und m eine ganze Zahl mit einem Wert von 2 bis 6 darstellen),
eine Gruppe der Formel $y_2$:

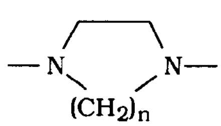

($y_2$)

(worin n die ganzen Zahlen 2 oder 3 darstellt),
eine Gruppe der Formel $y_3$:

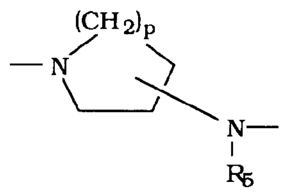

(y3)

(worin p Null oder die ganzen Zahlen 1 oder 2 und

- $R_5$ ein Wasserstoffatom, eine Alkylgruppe mit 1 bis 5 Kohlenstoffatomen oder eine Cycloalkylgruppe mit 3 bis 7 Kohlenstoffatomen darstellen),
  oder eine Gruppe der Formel $y_4$:

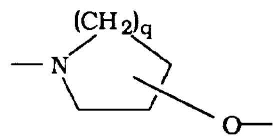

(y4)

(in der q Null oder die ganzen Zahlen 1 oder 2 darstellt)

- $R_3$ eine Diphenylmethylgruppe, die gegebenenfalls an den Benzolringen durch eines oder mehrere Halogenatome substituiert ist,

eine Gruppe der Formel $z_1$:

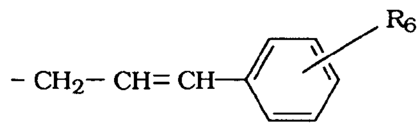

($z_1$)

(worin $R_6$ ein Wasserstoffatom oder ein Halogenatom darstellt),
eine Gruppe der Formel $z_2$:

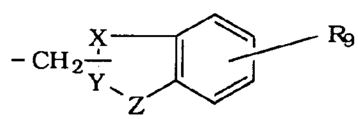

($z_2$)

(worin X-Y- einen Rest der Formel

$$- CH = CH - (CR_7 R_8)_r -$$

darstellen, worin r Null oder 1 und $R_7$ und $R_5$, die gleichartig oder verschieden sein können, jeweils ein Wasserstoffatom oder eine Methylgruppe, Z ein Sauerstoffatom oder ein Schwefelatom und $R_9$ ein Wasserstoffatom oder ein Halogenatom darstellen) bedeuten,
sowie deren mit einer therapeutisch annehmbaren anorganischen oder organischen Säure erhaltenen Additionssalze zur Herstellung von Arzneimitteln, welche die gegen Antikrebsmittel und Antimalariamittel erworbene Resistenz aufeheben.

**Patentanspruch für folgenden Vertragsstaat : ES**

1. Verwendung von Verbindungen der Formel I:

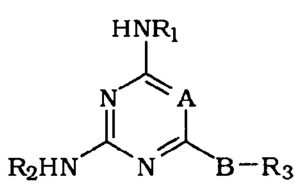

(I)

in der

- A eine Gruppe -CH- oder ein Stickstoffatom,
- $R_1$ und $R_2$, die gleichartig oder verschieden sein können, jeweils eine geradkettige oder verzweigte Kohlenwasserstoffgruppe mit 3 bis 5 Kohlenstoffatomen, die gegebenenfalls eine oder zwei Doppelbindungen aufweisen und gegebenenfalls durch eine oder mehrere Hydroxylgruppen substituiert sein kann,
- B eine Gruppe der Formel $y_1$:

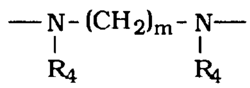

($y_1$)

(worin $R_4$ ein Wasserstoffatom oder eine Alkylgruppe mit 1 bis 5 Kohlenstoffatomen und m eine ganze Zahl mit einem Wert von 2 bis 6 darstellen),
eine Gruppe der Formel $y_2$:

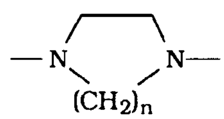

($y_2$)

(worin n die ganzen Zahlen 2 oder 3 darstellt),
eine Gruppe der Formel $y_3$:

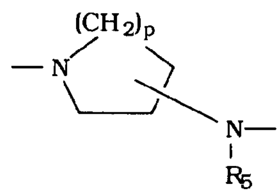

($y_3$)

(worin p Null oder die ganzen Zahlen 1 oder 2 und

- $R_5$ ein Wasserstoffatom, eine Alkylgruppe mit 1 bis 5 Kohlenstoffatomen oder eine Cycloalkylgruppe mit 3 bis 7 Kohlenstoffatomen darstellen),
oder eine Gruppe der Formel $y_4$:

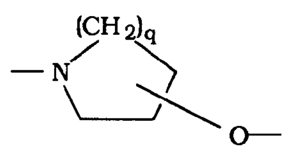

($y_4$)

(in der q Null oder die ganzen Zahlen 1 oder 2 darstellt)

- $R_3$ eine Diphenylmethylgruppe, die gegebenenfalls an den Benzolringen durch eines oder mehrere Halogenatome substituiert ist,

eine Gruppe der Formel $z_1$:

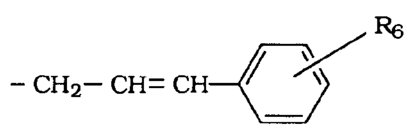

($z_1$)

(worin $R_6$ ein Wasserstoffatom oder ein Halogenatom darstellt),

eine Gruppe der Formel $z_2$:

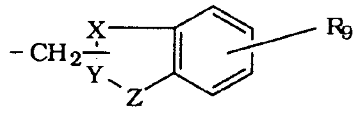

($z_2$)

(worin X-Y- einen Rest der Formel

$$- CH = CH - (CR_7R_8)_r -$$

darstellen, worin r Null oder 1 und $R_7$ und $R_8$, die gleichartig oder verschieden sein können, jeweils ein Wasserstoffatom oder eine Methylgruppe, Z ein Sauerstoffatom oder ein Schwefelatom und $R_9$ ein Wasserstoffatom oder ein Halogenatom darstellen) bedeuten, sowie deren mit einer therapeutisch annehmbaren anorganischen oder organischen Saure erhaltenen Additionssalze zur Herstellung von Arzneimitteln, welche die gegen Antikrebsmittel und Antimalariamittel erworbene Resistenz aufeheben.

**Patentanspruch für folgenden Vertragsstaat : GR**

1. Verwendung von Verbindungen der Formel I:

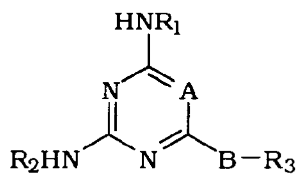

(I)

in der

- A eine Gruppe -CH- oder ein Stickstoffatom,
- $R_1$ und $R_2$, die gleichartig oder verschieden sein können, jeweils eine geradkettige oder verzweigte Kohlenwasserstoffgruppe mit 3 bis 5 Kohlenstoffatomen, die gegebenenfalls eine oder zwei Doppelbindungen aufweisen und gegebenenfalls durch eine oder mehrere Hydroxylgruppen substituiert sein kann,
- B eine Gruppe der Formel $y_1$:

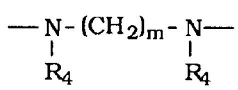

($y_1$)

(worin $R_4$ ein Wasserstoffatom oder eine Alkylgruppe mit 1 bis 5 Kohlenstoffatomen und m eine ganze Zahl mit einem Wert von 2 bis 6 darstellen),
eine Gruppe der Formel $y_2$:

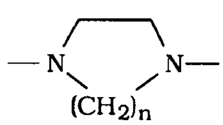

($y_2$)

(worin n die ganzen Zahlen 2 oder 3 darstellt),
eine Gruppe der Formel $y_3$:

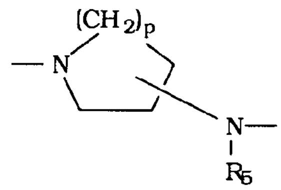

(y3)

(worin p Null oder die ganzen Zahlen 1 oder 2 und

- $R_5$ ein Wasserstoffatom, eine Alkylgruppe mit 1 bis 5 Kohlenstoffatomen oder eine Cycloalkylgruppe mit 3 bis 7 Kohlenstoffatomen darstellen), oder eine Gruppe der Formel $y_4$:

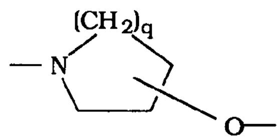

(y4)

(in der q Null oder die ganzen Zahlen 1 oder 2 darstellt)

- $R_3$ eine Diphenylmethylgruppe, die gegebenenfalls an den Benzolringen durch eines oder mehrere Halogenatome substituiert ist,

eine Gruppe der Formel $z_1$:

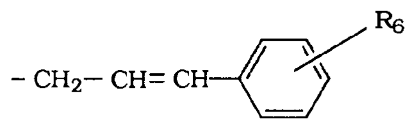

(z1)

(worin $R_6$ ein Wasserstoffatom oder ein Halogenatom darstellt), eine Gruppe der Formel $z_2$:

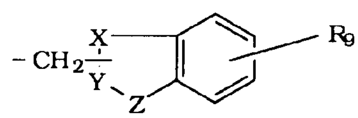

(z2)

(worin X-Y- einen Rest der Formel

$$- CH = CH - (CR_7 R_8)_r -$$

darstellen, worin r Null oder 1 und $R_7$ und $R_5$, die gleichartig oder verschieden sein können, jeweils ein Wasserstoffatom oder eine Methylgruppe, Z ein Sauerstoffatom oder ein Schwefelatom und $R_9$ ein Wasserstoffatom oder ein Halogenatom darstellen) bedeuten,
sowie deren mit einer therapeutisch annehmbaren anorganischen oder organischen Säure erhaltenen Additionssalze zur Herstellung von Arzneimitteln, welche die gegen Antikrebsmittel und Antimalariamittel erworbene Resistenz aufeheben.

## Claims

### Claims for the following Contracting States : AT, BE, CH, DE, DK, FR, GB, IT, LI, LU, NL, SE

**1.** Use, for the preparation of medicaments that reverse acquired resistance to anti-cancer and anti-malarial agents, of compounds of formula I:

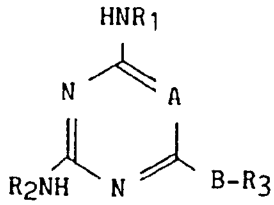

(I)

in which

- **A** represents a group -CH- or a nitrogen atom,
- **$R_1$ and $R_2$**, which are identical or different, each represents a straight-chain or branched hydrocarbon radical having from 3 to 5 carbon atoms which optionally contains one or two double bonds and is optionally substituted by one or more hydroxy radicals,
- **B** represents a radical of the formula $y_1$:

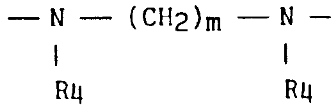

($y_1$)

(in which $R_4$ represents a hydrogen atom or an alkyl radical having from 1 to 5 carbon atoms and m represents an integer from 2 to 6),
a radical of the formula $y_2$:

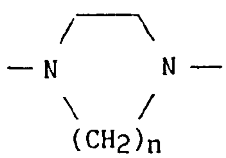

($y_2$)

(in which n represents an integer 2 or 3),
a radical of the formula $y_3$:

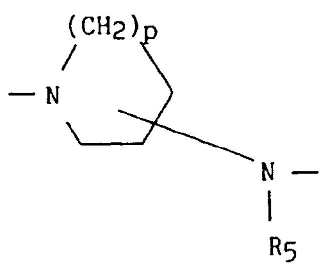

($y_3$)

(in which p represents zero or an integer 1 or 2, and $R_5$ represents a hydrogen atom, an alkyl radical having from 1 to 5 carbon atoms, or a cycloalkyl radical having from 3 to 7 carbon atoms),
or a radical of the formula $y_4$:

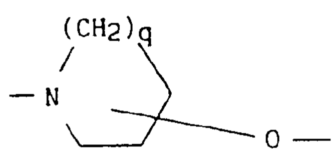

($y_4$)

(in which q represents zero or an integer 1 or 2),

- **$R_3$** represents a diphenylmethyl radical optionally substituted on the benzene rings by one or more halogen atoms,

a radical of the formula $z_1$:

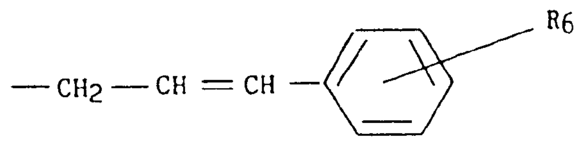

($z_1$)

(in which $R_6$ represents a hydrogen atom or a halogen atom),
or a radical of the formula $z_2$:

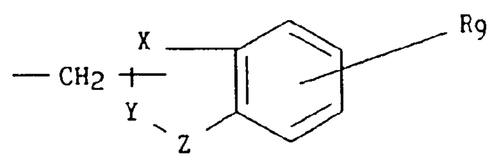

($z_2$)

(in which X-Y- represents a radical of the formula

$$-CH=CH-(CR_7R_8)_r- ,$$

in which r represents 0 or 1 and $R_7$ and $R_8$, which are identical or different, each represents a hydrogen atom or a methyl radical,
Z represents an oxygen or sulphur atom and $R_9$ represents a hydrogen atom or a halogen atom),
and their addition salts obtained with a therapeutically compatible mineral or organic acid.

**Claims for the following Contracting State : ES**

**1.** Use, for the preparation of medicaments that reverse acquired resistance to anti-cancer and anti-malarial agents, of compounds of formula I:

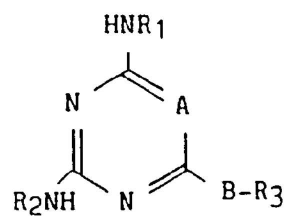

(I)

in which

- **A** represents a group -CH- or a nitrogen atom,
- **$R_1$ and $R_2$**, which are identical or different, each represents a straight-chain or branched hydrocarbon radical having from 3 to 5 carbon atoms which optionally contains one or two double bonds and is optionally substituted by one or more hydroxy radicals,
- **B** represents a radical of the formula $y_1$:

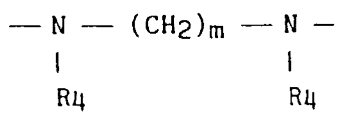

($y_1$)

(in which $R_4$ represents a hydrogen atom or an alkyl radical having from 1 to 5 carbon atoms and m represents an integer from 2 to 6),
a radical of the formula $y_2$:

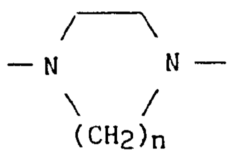

($y_2$)

(in which n represents an integer 2 or 3), a radical of the formula $y_3$:

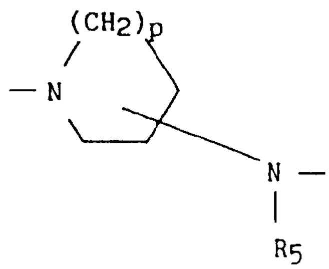

($y_3$)

(in which p represents zero or an integer 1 or 2, and $R_5$ represents a hydrogen atom, an alkyl radical having from 1 to 5 carbon atoms, or a cycloalkyl radical having from 3 to 7 carbon atoms), or a radical of the formula $y_4$:

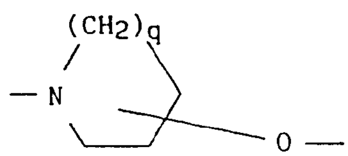

($y_4$)

(in which q represents zero or an integer 1 or 2),

- $R_3$ represents a diphenylmethyl radical optionally substituted on the benzene rings by one or more halogen atoms,

a radical of the formula $z_1$:

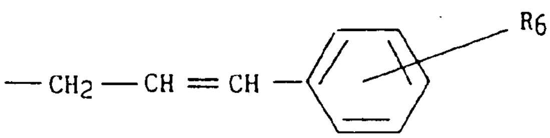

($z_1$)

(in which $R_6$ represents a hydrogen atom or a halogen atom), or a radical of the formula $z_2$:

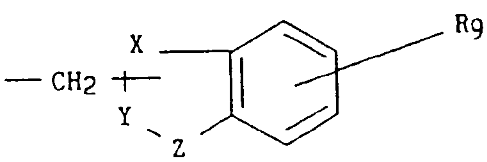

($z_2$)

(in which X-Y- represents a radical of the formula

$$-CH=CH-(CR_7R_8)_r- ,$$

in which r represents 0 or 1 and $R_7$ and $R_8$, which are identical or different, each represents a hydrogen atom or a methyl radical,

Z represents an oxygen or sulphur atom and $R_9$ represents a hydrogen atom or a halogen atom), and their addition salts obtained with a therapeutically compatible mineral or organic acid.

**Claims for the following Contracting State : GR**

**1.** Use, for the preparation of medicaments that reverse acquired resistance to anti-cancer and anti-malarial agents, of compounds of formula I:

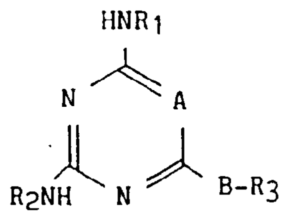

(I)

in which

- **A** represents a group -CH- or a nitrogen atom,
- **$R_1$ and $R_2$**, which are identical or different, each represents a straight-chain or branched hydrocarbon radical having from 3 to 5 carbon atoms which optionally contains one or two double bonds and is optionally substituted by one or more hydroxy radicals,
- **B** represents a radical of the formula $y_1$:

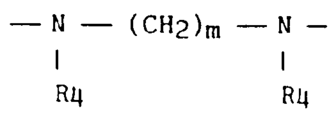

($y_1$)

(in which $R_4$ represents a hydrogen atom or an alkyl radical having from 1 to 5 carbon atoms and m represents an integer from 2 to 6),
a radical of the formula $y_2$:

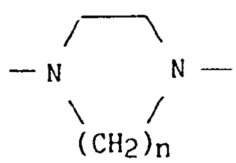

($y_2$)

(in which n represents an integer 2 or 3),
a radical of the formula $y_3$:

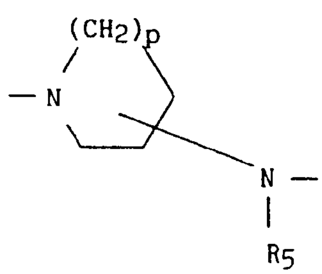

($y_3$)

(in which p represents zero or an integer 1 or 2, and $R_5$ represents a hydrogen atom, an alkyl radical having from 1 to 5 carbon atoms, or a cycloalkyl radical having from 3 to 7 carbon atoms),
or a radical of the formula $y_4$:

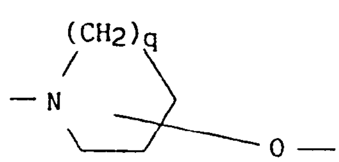

($y_4$)

(in which q represents zero or an integer 1 or 2),

- **$R_3$** represents a diphenylmethyl radical optionally substituted on the benzene rings by one or more halogen atoms,

a radical of the formula $z_1$:

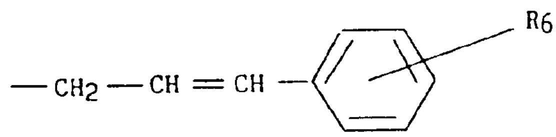

($z_1$)

(in which $R_6$ represents a hydrogen atom or a halogen atom),
or a radical of the formula $z_2$:

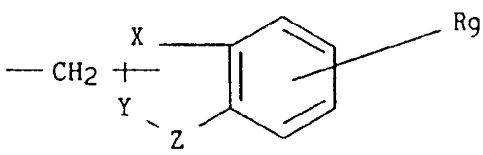

($z_2$)

(in which X-Y- represents a radical of the formula

$$-CH=CH-(CR_7R_8)_r- ,$$

in which r represents 0 or 1 and $R_7$ and $R_8$, which are identical or different, each represents a hydrogen atom or a methyl radical,
Z represents an oxygen or sulphur atom and $R_9$ represents a hydrogen atom or a halogen atom),
and their addition salts obtained with a therapeutically compatible mineral or organic acid.